# EUROPÄISCHE PATENTSCHRIFT

(11) **EP 0 401 778 B1**
(45) Veröffentlichungstag und Bekanntmachung des Hinweises auf die Patenterteilung: **27.07.1994**
(21) Anmeldenummer: 90110682.3
(22) Anmeldetag: 06.06.1990
(51) Int. Cl.: A61B 19/00, A61B 1/00

(54) **Endoskop, insbesondere Resektoskop**
Endoscope, in particular resectoscope
Endoscope, en particulier resectoscope

(30) Priorität: 08.06.1989 DE 3918719
(43) Veröffentlichungstag der Anmeldung: 12.12.1990
(73) Patentinhaber: Richard Wolf GmbH, 75438 Knittlingen (DE)
(72) Erfinder: Bonnet, Ludwig, D-7134 Knittlingen (DE); Wetterauer, Ulrich, D-7800 Freiburg (DE)
(74) Vertreter: Wilcken, Hugo

(56) Entgegenhaltungen:
- US-A- 1 679 950
- US-A- 4 327 716
- US-A- 4 834 068
- US-A- 4 848 322

## Beschreibung

Die Erfindung geht aus von einem Endoskop, insbesondere Resektoskop, zum Resizieren von Gewebe gemäß dem Oberbegriff des Patentanspruches 1. Ein derartiges Endoskop mit einem Schutzschild ist durch US-A-4 834 068 bekannt.

Bei derartigen, beispielsweise bei der Prostataresektion verwendeten Resektoskopen werden üblicherweise größere Mengen Spülflüssigkeit benötigt, um insbesondere die resizierten Gewebeteile abzuführen. Dabei wird so vorgegangen, daß nach erfolgter Resektion einer bestimmten Gewebemenge der Arbeitseinsatz aus dem in die Körperhöhle eingeführten Resektoskopschaft herausgezogen wird, um durch den relativ großen Schaftquerschnitt die resizierten Gewebeteile und die Spülflüssigkeit aus der Körperhöhle herauszuführen.

Bei der Handhabung des Resektoskopes ist es in der Regel unvermeidbar, daß Spülflüssigkeit in das Gesicht des den endoskopischen Eingriff durchführenden und über die Endoskopoptik verfolgenden Operateurs gelangt, da man die Spülflüssigkeit an dem proximalseitigen Ende des Resektoskopschaftes in einen geeigneten Auffangbehälter frei ablaufen läßt. Ein derartiger Kontakt mit der Spülflüssigkeit wird von dem Operateur nicht nur als sehr störend und unangenehm empfunden, sondern es ist damit auch die große Gefahr verbunden, daß durch diesen Kontakt beispielsweise Aids- und/oder Hepatitiserreger übertragen werden können, die über Schleimhäute von Augen, Nase und Mund in das Blut gelangen und die jeweilige Krankheit auslösen können.

Um derartige Infektionen zu vermeiden bzw. das Infektionsrisiko zu reduzieren, sind transparente, vor dem Gesicht zu tragende Schutzmasken in Anwendung. Das Tragen solcher Gesichtsmasken wird jedoch aufgrund des relativ geringen Tragekomforts sowie der schlechten Hinterlüftung als sehr störend empfunden. Schutzbrillen und Mundschutz tragen ebenfalls zur Reduzierung des Infektionsrisikos bei, können dieses jedoch ebensowenig vollständig ausschließen. Im übrigen sind auch derartige Hilfsmittel unbeliebt, da sie als bei der Durchführung des Eingriffes störend empfunden werden.

Eine fortschrittlichere Lösung zeigt die vorerwähnte US-A-4 834 068. Diese Lösung sieht einen mit einem Endoskop verbindbaren, kreisförmigen Schutzschild mit einem zentralen Durchbruch vor, der an seinem inneren Umfang einen elastischen Ring aufweist, durch den eine Öffnung mit variablem Durchmesser gebildet wird. Zur Befestigung auf dem Endoskop wird der Schutzschild über die proximalen Teile des Endoskopes gestreift, wobei der elastische Ring entsprechend gedehnt und schließlich aufgrund seiner Rückstellkraft an der vorgesehenen Stelle festgehalten wird. Das zwecks Reinigung erforderliche, mit ständigen Dehnungen verbundene Abnehmen und anschließende Wiederaufsetzen des Schutzschildes ist umständlich, und die Haltbarkeit des elastischen Ringes bzw. des Schutzschildes ist durch die ständigen Dehnungen begrenzt.

Es ist die Aufgabe der Erfindung, den einen endoskopischen Eingriff ausführenden Operateur vor der Gefahr einer Infektion durch umherspritzende Spülflüssigkeit sicher zu schützen, ohne daß dieser hinderliche oder sonstwie störende Hilfsmittel anlegen muß.

Diese Aufgabe wird durch die Kennzeichenmerkmale des Patentanspruches 1 gelöst.

Die damit erzielbaren Vorteile bestehen neben den Vorteilen, daß der Schutzschild und das Resektoskop eine Einheit bilden und das Gesicht des den Eingriff über die Resektoskopoptik verfolgenden Operateurs gegenüber dem Wirkungsbereich des Arbeitseinsatzes unmittelbar abgedeckt ist, insbesondere darin, daß sich der Schutzschild sehr einfach durch Aufstecken montieren läßt und zwar wegen der beiden Schenkel, die durch einen Einschnitt gebildet sind, dessen Breite dem Durchmesser des den Schutzschild aufnehmenden Instrumenten- oder Optikteiles im wesentlichen entspricht.

Ein besonders sicherer Halt kann dabei dadurch erreicht werden, daß der Schutzschild eine zentrale Ausnehmung aufweist, deren Durchmesser etwas geringer als der Durchmesser des den Schutzschild aufnehmenden Instrumenten- oder Optikteiles ist, wobei die Ausnehmung in einen Einschnitt übergeht, dessen Breite kleiner ist als der Durchmesser der vorerwähnten Ausnehmung.

Nach einer weiteren Ausgestaltung der Erfindung sind der die zentrale Ausnehmung umschließende Teil des Schutzschildes aus einem starren, elastisch verformbaren Werkstoff und die den Einschnitt begrenzenden Teile aus dem gleichen oder aus einem weicheleastischen Werkstoff, vorzugsweise jeweils in transparenter Ausführung, gefertigt. Die weichelastische Ausbildung der sich an den starren Teil anschließenden Schenkel wirkt sich vorteilhaft bei der Handhabung des Resektoskopes aus, da diese sich an die Handoberfläche anlegen und jeder Handbewegung des Operateurs folgen.

Durch den sich an den oberen Teil der Ausnehmung anschließenden Einschnitt können der Schlitz und die Ausnehmung in Grenzen federelastisch verformt werden, so daß der Schutzschild durch die aus der Verformung resultierende Federvorspannung sicher auf dem Instrumenteil oder der Optik festgelegt werden kann.

Gemäß einer anderen Ausgestaltung kann der Schutzschild im Bereich der Ausnehmung einen Kupplungsteil aufweisen, so daß beispielsweise eine Anordnung bzw. Festlegung zwischen der Endoskopoptik und dem Arbeitseinsatz möglich ist.

Die Erfindung ist nachfolgend anhand eines in der Zeichnung dargestellten Ausführungsbeispieles näher erläutert.
**Es zeigen:**
- Figur 1: ein Resektoskop mit einem Schutzschild,
- Figur 2: den erfindungsgemäßen Schutzschild in Aufsicht und vergrößert dargestellt,
- Figur 3: den Schutzschild nach Figur 2 in Seitenansicht.

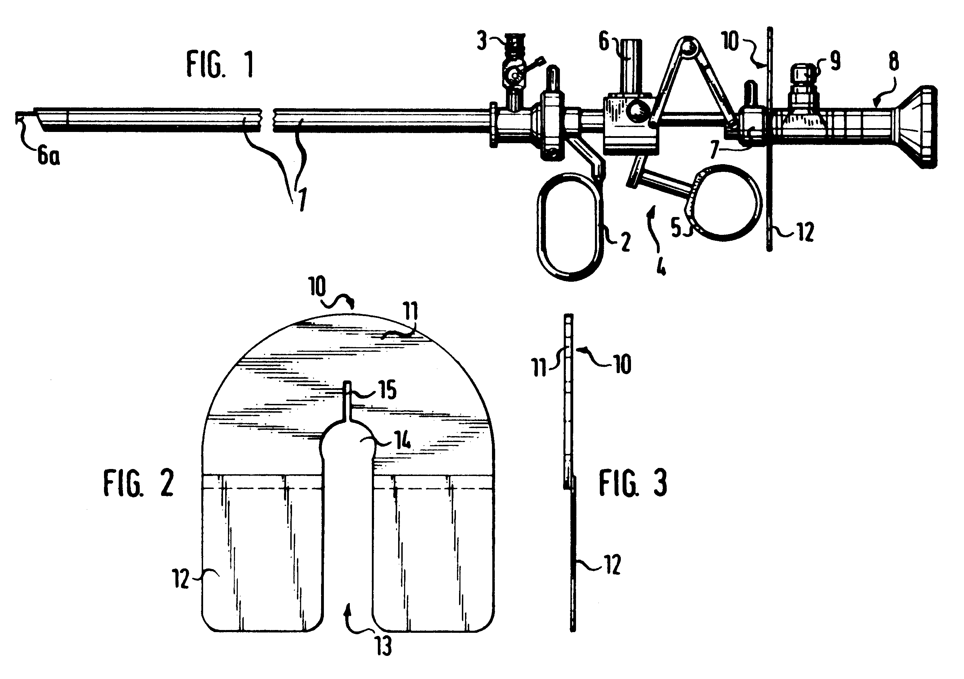

Die Figur 1 gibt ein Endoskop in Form eines Resektoskops wieder, wie es in der Praxis eingesetzt wird. Es umfaßt im wesentlichen einen Resektoskopschaft 1 mit einer Handhabe 2 und einem Zulaufstutzen 3 für die Spülflüssigkeit. Der Resektoskopschaft 1 dient der Aufnahme und Hindurchführung eines bekannten Arbeitseinsatzes 4 mit einem Daumenring 5 und einem Hochfrequenz-Stromanschluß 6 für eine Resektionsschlinge 6a sowie einer bekannten, mittels einer proximalseitigen Kupplung 7 lösbar mit dem Arbeitseinsatz 4 verbundenen Endoskopoptik 8 mit einem Lichtleiteranschluß 9.

Ein erfindungsgemäßer Schutzschild 10 ist in dem gezeigten Ausführungsbeispiel vorzugsweise zwischen dem Lichtleiteranschluß 9 der Endoskopoptik 8 und der Kupplung 7 angeordnet. Wie in Figur 2 erkennbar ist, weist der Schutzschild 10 einen Stegteil 11 aus einem starren, elastisch verformbaren Werkstoff auf, an den sich weichelastisch ausgebildete oder aus dem gleichen Werkstoff bestehende Schenkel 12 unlösbar befestigt anschließen, die dem Schutzschild 10 eine U-förmige Gestalt geben, wobei die Schenkel 12 einen Einschnitt 13 zwischen sich begrenzen. Im Zentrum des Schutzschildes 10 befindet sich eine Ausnehmung 14, an deren oberem Ende ein radialer Entlastungsschlitz 15 vorgesehen ist, dessen Dimension so gewählt ist, daß die auf den Schutzschild 10 beim Aufstecken auftretende Verformung im elastischen Bereich verbleibt, so daß die daraus resultierende Rückstellkraft eine sichere Halterung des Schutzschildes 10 auf dem entsprechenden Instrumententeil bewirkt.

In weiterer, nicht gezeigter Ausbildung des erfindungsgemäßen Schutzschildes 10 ist es denkbar, für die lösbare Befestigung desselben auf dem Arbeitseinsatz 4 oder der Endoskopoptik 8 Befestigungsmittel vorzusehen, die eine form- oder kraftschlüssige Fixierung ermöglichen. Auch ein Kupplungsteil kann vorgesehen sein. Solche Kupplungsteile haben gegenüber der eingangs beschriebenen Ausführugnsform des Schutzschildes 10 den Vorteil der besseren Fixierung zwischen den jeweiligen Instrumententeilen.

Es ist ferner denkbar, zur Optimierung des Schutzeffektes die Größe oder Raumform des Schutzschildes 10 in geeigneter Weise anzupassen. Dabei kann dieser auch in Richtung zum Patienten hin gekrümmt sein.

## Patentansprüche

1. Endoskop, insbesondere Resektoskop, zum Resizieren von Gewebe, mit einem in eine Körperhöhle einfuhrbaren Endoskopschaft (1) zum Zu- und Abführen einer Spülflussigkeit und zur Aufnahme eines an seinem proximalen Ende betätigbaren Arbeitseinsatzes (4), dessen Wirkungsbereich über eine an seinem proximalen Ende angeordnete Endoskopoptik (8) visuell kontrollierbar ist, wobei ein Schutzschild (10) auf einem Längenabschnitt des Endoskopes lösbar befestigbar ist, welcher Längenabschnitt aus den aus dem Endoskopschaft (1) proximal herausragenden Abschnitten des Arbeitseinsatzes (4) und der Endoskopoptik (8) besteht, dadurch gekennzeichnet, daß der Schutzschild (10) von U-förmiger Gestalt ist, wobei die beiden Schenkel (12) durch einen Einschnitt (13) gebildet sind, dessen Breite geringer als der Durchmesser des den Schutzschild (10) aufnehmenden Einsatzes (4) oder der Optik (8) ist.

2. Endoskop nach Anspruch 1, dadurch gekennzeichnet, daß der Schutzschild (10) eine zentrale Ausnehmung (14) aufweist, deren Durchmesser etwas geringer ist als der Durchmesser des den Schutzschild aufnehmenden Einsatzes (4) oder der Optik (8), wobei die Ausnehmung (14) in den Einschnitt (13) übergeht.

3. Endoskop nach Anspruch 1 oder 2, gekennzeichnet durch einen sich an die zentrale Ausnehmung (14) des Schutzschildes (10) anschließenden Schlitz (15), um den die beiden Schenkel (12) verbindenden Stegteil (11) des Schutzschildes elastisch zu machen.

4. Endoskop nach Anspruch 1, dadurch gekennzeichnet, daß der Schutzschild (10) zwischen einer Kupplung (7) des Arbeitseinsatzes (4) und einem Lichtleiteranschluß (9) der Endoskopoptik (8) lösbar festlegbar ist.

5. Endoskop nach einem der Ansprüche 1 bis 4, dadurch gekennzeichnet, daß der Stegteil (11) des Schutzschildes (10) aus einem starren Werkstoff und die beiden mit dem Stegteil verbundenen Schenkel (12) des Schutzschildes aus weichelastischem Werkstoff bestehen und daß alle Teile (11, 12) des Schutzschildes transparent sind.

## Claims

1. Endoscope, in particular resectoscope, for resecting tissue, having an endoscope shaft (1) which can he introduced into a body cavity for supply and removal of a rinsing fluid and for receiving a working insert (4) which can be actuated at its proximal end, it being possible for the effective region of the working insert (4) to be visually monitored by means of an endoscope optical system (8) arranged at its proximal end, it being possible for a protective shield (10) to be releasably attached to one longitudinal section of the endoscope, which longitudinal section comprises the sections of the working insert (4) projecting proximally from the endoscope shaft (1) and the endoscope optical system (8), characterised in that the protective shield (10) has a U-shaped design, the two limbs (12) being formed by a slot (13), the width of which is smaller than the diameter of the insert (4) receiving the protective shield (10) or the optical system (8).

2. Endoscope according to claim 1, characterised in that the protective shield (10) has a central recess (14), the diameter of which is somewhat smaller than the diameter of the insert (4) receiving the protective shield or of the optical system (8), the recess (14) changing into the slot (13).

3. Endoscope according to claim 1 or 2, characterised by a slit (15) adjoining the central recess (14) of the protective shield (10) in order to render resilient the leg section (11) of the protective shield connecting the two limbs (12).

4. Endoscope according to claim 1, characterised in that the protective shield (10) can be releasably fixed between a coupling (7) of the working insert (4) and a light-guide connection (9) of the endoscope optical system (8).

5. Endoscope according to one of claims 1 to 4, characterised in that the leg section (11) of the protective shield (10) consists of a rigid material and the two limbs (12) of the protective shield connected to the leg section consist of soft-resilient material, and in that all parts (11, 12) of the protective shield are transparent.

## Revendications

1. Endoscope, notamment résecteur endoscopique, destiné à la résection de tissus, comportant une tige d'endoscope (1) pouvant être introduite dans une cavité du corps et destinée à amener et à évacuer un liquide de rinçage, ainsi qu'à recevoir un insert de travail (4) pouvant être actionné à son extrémité proximale, et dont la zone d'action peut être contrôlée visuellement par l'intermédiaire d'une optique d'endoscope (8) disposée à son extrémité proximale, un écran de protection (10) pouvant être fixé de manière amovible sur un tronçon de longueur de l'endoscope, ce tronçon de longueur étant constitué par les tronçons de l'insert de travail (4) sortant, côté proximal, de la tige d'endoscope (1), et de l'optique d'endoscope (8), caractérisé en ce que l'écran de protection (10) présente une configuration en forme de "U", les deux branches (12) étant formées par une entaille (13) dont la largeur est inférieure au diamètre de l'insert (4) ou de l'optique (8), qui reçoit l'écran de protection (10).

2. Endoscope selon la revendication 1, caractérisé en ce que l'écran de protection (10) présente un évidement central (14), dont le diamètre est légèrement inférieur au diamètre de l'insert (4) ou de l'optique (8), qui reçoit l'écran de protection, l'évidement (14) s'ouvrant sur l'entaille (13).

3. Endoscope selon la revendication 1 ou 2, caractérisé par une fente (15) se raccordant à l'évidement central (14) de l'écran de protection (10), en vue de rendre élastique la partie de barrette (11) de l'écran de protection, qui relie les deux branches (12).

4. Endoscope selon la revendication 1, caractérisé en ce que l'écran de protection (10) peut être fixé de manière amovible, entre un raccord d'accouplement (7) de l'insert de travail (4) et un raccord de connexion de guide de lumière (9) de l'optique d'endoscope (8).

5. Endoscope selon l'une des revendications 1 à 4, caractérisé en ce que la partie de barrette (11) de l'écran de protection (10) est réalisée en un matériau rigide, et les deux branches (12) de l'écran de protection, reliées à la partie de barrette, sont réalisées en un matériau souple et élastique, et en ce que toutes les parties (11, 12) de l'écran de protection sont transparentes.
